# EUROPEAN PATENT SPECIFICATION

(11) **EP 1 071 411 B1**
(45) Date of publication and mention of the grant of the patent: **14.03.2007**
(21) Application number: 98918392.6
(22) Date of filing: 20.04.1998
(51) Int. Cl.: A61K 31/12, A61K 31/125, A61P 37/04

(54) **TOPICAL IMMUNOSTIMULATION TO INDUCE LANGERHANS CELL MIGRATION**
TOPISCHE IMMUNOSTIMULIERUNG ZUR INDUZIERUNG DER MIGRATION DER LANGERHANSZELLEN
IMMUNOSTIMULATION TOPIQUE VISANT A INDUIRE LA MIGRATION DES CELLULES DE LANGERHANS

(43) Date of publication of application: 31.01.2001
(73) Proprietor: TORREY PINES INSTITUTE FOR MOLECULAR STUDIES, San Diego, CA 92121 (US)
(72) Inventor: COWING, Carol, O., La Jolla, CA 92037 (US)
(74) Representative: Van Malderen, Joëlle
(86) International application number: PCT/US1998/007817
(87) International publication number: WO 1999/053912

(56) References cited:
- EP-A- 0 308 210
- GB-A- 2 145 931
- US-A- 4 353 896
- US-A- 4 455 142
- US-A- 5 278 263
- US-A- 5 487 897
- US-A- 5 654 312
- A.H.ENK AND ALL.: "An essential role of Langerhans cell-derived IL-1beta in the initiation of primary immune response in skin" JOURNAL OF IMMUNOLOGY, vol. 150, 1993, pages 3698-3704, XP002299996

## Description

### Background of the Invention

The body's first line of defense against pathogens is the skin. The outermost layer, the stratum corneum, is a broad zone of 20 to 30 cell layers thick. The dead cell remnants which comprise the stratum corneum are almost completely filled with keratin fibrils and surrounded by highly ordered lipid bilayers. As long as the epidermis is unbroken, the heavily keratinized stratum corneum presents a formidable physical barrier to entry for most foreign substances. The mucous membranes which line the digestive, respiratory, urinary and reproductive tracts, provide a similar, but less formidable physical barrier, lacking the thick stratum corneum.

The underlying layers of the epithelium in both skin and mucous membranes are richly populated with immature dendritic cells, called Langerhans cells. These phagocytic leukocytes are poised for capture of antigens which may enter the lower strata of the epidermis through physical breaches in the stratum corneum. After physical trauma to the skin, signals are generated that induce Langerhans cells to leave the epithelium and migrate through afferent lymphatics to lymph nodes, carrying with them any antigens which had penetrated the protective stratum corneum (i.e. viral, bacterial, parasitic, allergic). Very small, lipophilic molecules, and some highly reactive molecules known as skin sensitizing agents, such as TNCB, poison ivy catechol, oxazolone, etc., may penetrate the intact stratum corneum, subsequently binding to proteins in the underlying epidermis and activating Langerhans cells.

Captured protein antigens are internalized and degraded by the Langerhans cells to yield small peptides which are incorporated into the peptide binding grooves of MHC molecules. The MHC-peptide complexes are then inserted into the plasma membranes for presentation to T cell receptors. During their migration to the lymph nodes, the Langerhans cells differentiate into mature lymphoid dendritic cells, losing their phagocytic properties and instead, expressing high levels of MHC class I and II molecules as well as costimulatory and adhesion molecules, essential for effective antigen presentation (Udey, *Clin*. *Exp*. *Immunol*. 107:6-8, 1997). While it is now well known that Langerhans cells migrate from the epithelium to the T cell areas of the draining lymph nodes, relatively little is known about the signals which induce migration and differentiation of the Langerhans cells. In any event, once in the lymph nodes, the differentiated Langerhans cells bearing MHC-peptide complexes activate primary CD4+ helper T cells and CD8+ cytolytic T cells. These newly differentiated Langerhans cells which are recent immigrants to the lymph nodes are the most potent inducers of T cell immunity known.

It has recently been demonstrated that mouse or human dendritic cells exposed to tumor antigens or peptides are effective inducers of tumor-specific immunity that can eliminate or suppress even established tumors (Young and Inaba, *J. Exp. Med.* 183:7-11, 1996; Zitvogel et al., *J. Exp. Med* 183:87-97, 1996; Celluzzi et al., *J. Exp. Med* 183:283.287, 1996; and Paglia et al., *J. Exp. Med* 183:317-322, 1996). In these studies, dendritic cells from bone marrow or blood were harvested, expanded in tissue culture, exposed to tumor antigens *in vitro,* and finally re-injected i.v. into the donor. Such individualized therapy is necessary in an outbred population to ensure that the appropriate, syngeneic MHC molecules are used for an individual's T cells and target tumor cells. While highly effective, this individualized procedure is too cumbersome, time consuming and costly to be a broadly applicable therapeutic measure.

### Summary of the Invention

A topical vaccination procedure is disclosed for enhancing an immune response against an antigen. Antigens from tumors, viral and bacterial pathogens, as well as parasites are encompassed within the scope of the present invention. The method involves administering the antigen in conjunction with: 1) a means for enhancing penetration of the antigen through the skin or mucous membranes, and 2) an agent for inducing Langerhans cell migration to the lymph nodes. The antigen is preferably a peptide of 3-20 amino acid residues in length, and more preferably, the peptide contains 8-14 amino acid residues. The antigen is preferably administered at a concentration range of about 1 µg/ml to about 100 mg/ml.

Means for enhancing penetration of the antigen include, use of a lipophilic vehicle or penetration enhancer, such as dimethylsulfoxide (DMSO) or azone, low frequency ultrasound, electroporation, iontophoresis, intraepidermal delivery, and combinations thereof. Preferably, peptide penetration of the stratum corneum is facilitated by dimethylsulfoxide in combination with one of the physical transdermal delivery means.

Agents which induce Langerhans cell migration are selected from the group consisting of dibutylphthalate, dibutyl-D-tartarate, N,N-diethyl-toluamide, dibutylfumarate, di(2-ethylhexyl)fumarate, diisooctylmaleate, diethylhexylmaleate, diisooctylfumarate, benzoic acid, benzalkoniumchloride, camphor, bihenylmaleate, dioctylphthalate, dibutylmaleate, dioctymaleate, dibutylsuccinate, dioctylsuccinate, dinonylphthalate, diisononylphthalate, dimethylphthalate, diethylphthalate, dipropylphthalate, diphenylphthalate, dibenzylbutylphthalate, and diethylmethylphthalate. Low frequency ultrasound may also be employed as an inducer of Langerhans cell migration. Preferably, dibutylphthalate is administered to induce Langerhans cell migration.

### Brief Description of the Drawings

Figure 1 illustrates the effect of the various treatment regimens on total and FITC+ lymphoid dendritic cells 2 days after treatment.
Figure 2 shows the kinetics of Langerhans cell migration in response to FITC in acetone and dibutylphthalate.
Figure 3 shows the effect of topical administration of FITC in acetone and dibutylphthalate on total lymph node dendritic cell number.
Figure 4 shows poor inhibition of EG7-OVA tumor growth by cutaneous topical administration of the tumor peptide SIINFEKL in acetone and dibutylphthalate.
Figure 5 illustrates the induction of complete EG7-OVA tumor-specific immunity by intravaginal topical application of SIINFEKL in acetone and dibutylphthalate.
Figure 6 shows complete inhibition of EG7-OVA tumor growth by cutaneous topical administration of SIINFEKL in DMSO, followed by dibutylphthalate in acetone.
Figure 7 shows the effect of various potential inducers of Langerhans cell migration in the FITC screening assay.

### Detailed Description of the Invention

According to the present invention, an antigen is administered topically for uptake by an individual's own Langerhans cells. A lipophilic solvent is preferably included to facilitate penetration of the antigen through the stratum corneum and into the underlying epidermis. In one embodiment of the present invention, a physical stimulus, such as low frequency ultrasound or an electric field, may be applied to the skin following the topical administration, in order to enhance antigen penetration through the stratum corneum. The administration of antigen to the skin is accompanied by the topical application of further agent(s), such as dibutylphthalate, which induce Langerhans cell migration to the draining lymph node.

### Induction of Langerhans Cell Migration by Dibutylphthalate

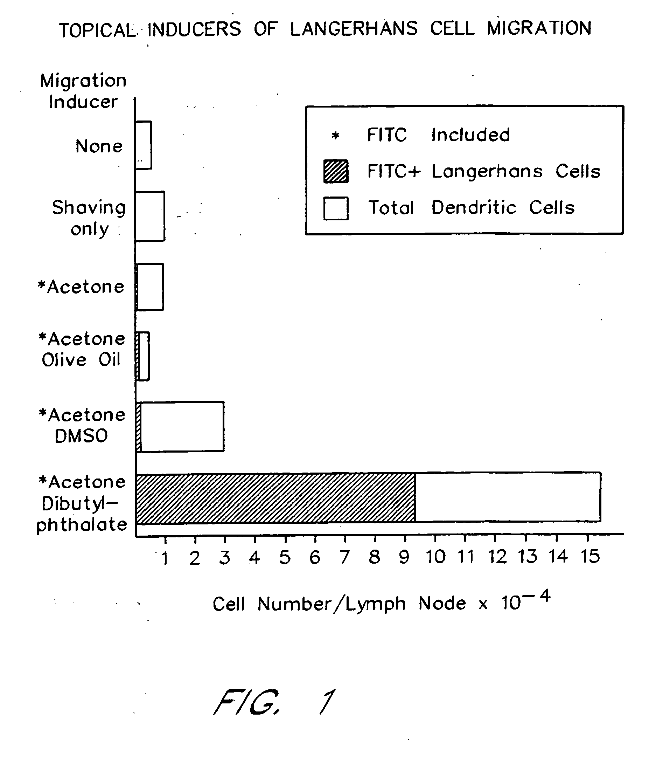

Fluorescein isothiocyanate (FITC) has been used extensively by the inventor and others, to characterize Langerhans cell migration. FITC is a small (mw 389) non-peptide, largely lipophilic molecule, capable of crossing the stratum corneum. Immunogenicity of FITC is due to the reactivity of its isothiocyanate group (-N-C-S) with free amino groups, permitting it to be covalently bound to proteins and/or peptides. C57BL/6 mice received the following treatments: 1) none; 2) abdomen shaving only; 3) topical administration of FITC in acetone; 4) topical administration of FITC in acetone and olive oil; 5) topical administration of FITC in acetone and DMSO diluted in phosphate buffered saline (PBS); and 6) topical administration of FITC in acetone and dibutylphthalate. The draining inguinal lymph nodes were then examined for immigrant Langerhans cells by immunofluorescent flow cytometry each day thereafter. Migratory Langerhans cells were identified by the presence of FITC and expression of high MHC class II molecules or by a Langerhans cell-specific monoclonal antibody (NLDC-145). Other dendritic cell residents of the lymph nodes could also be differentiated using a dendritic cell-specific monoclonal antibody, 33D1. The uptake of FITC and the Langerhans cell response to migratory signals were observable in the draining lymph node as early as six hours after topical administration. However, 48-72 hours was required for maximal rate of immigration into the lymph node. Figure 1 illustrates the effect of the various treatment regimens on total and FITC + lymphoid dendritic cells 2 days after treatment. In animals administered FITC in acetone, there was no enhancement in FITC+ Langerhans cell number induced by olive oil or dimethylsulfoxide (DMSO), although DMSO had a modest stimulatory effect on nonspecific immigration, suggesting that antigen contact and Langerhans cell migration are independently regulated. The addition of dibutylphthalate had a striking effect on both FITC+ and total dendritic cell migration into the lymph node. Thus, dibutylphthalate was a very potent migration inducer.

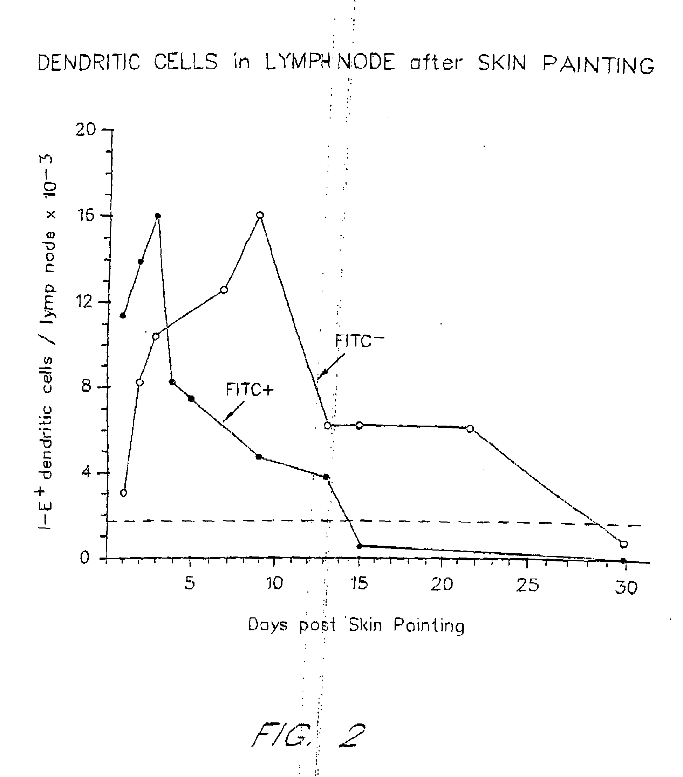

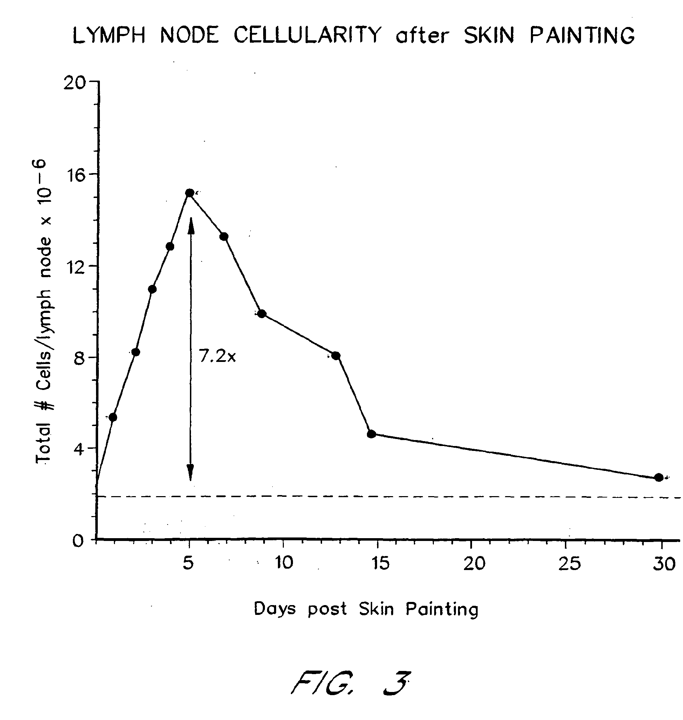

In mice treated with FITC in acetone and dibutylphthalate, the kinetics of Langerhans cell migration is shown in Figure 2. High numbers of FITC+ Langerhans cells were present in the lymph node by 12 hours and the peak frequency of immigrant FITC+ Langerhans cells occurred about 2 to 3 days following topical administration. Note however, that in addition to inducing the migration of FITC+ Langerhans cells, the number of unlabeled dendrite cells was also markedly enhanced, reaching peak levels 9-10 days following treatment. The unlabeled dendritic cells were most likely Langerhans cells that had lost the FITC label. Total lymph node cells in response to the topical administration is shown in Figure 3. Five days after skin painting, the lymph node cell population had increased by approximately 7.2 fold over baseline levels. These results indicate that antigen (FITC) in acetone and dibutylphthalate not only stimulated antigen+ Langerhans cell migration, but also resulted in a dramatic T and B cell response in the draining lymph node.

### Induction of Langerhans Cell-Mediated Tumor-Specific Immunity

The C57BL/6 thymoma tumor cell line, EL4, was transfected with the complete gene for chicken ovalbumin (OVA). The resulting transfected cell line, EG7-OVA, expresses chicken ovalbumin. An eight amino acid peptide, OVA 257-264, with the sequence, SIINFEKL, is expressed in the MHC class I molecule K^{b}, where it has been demonstrated to function as a tumor-associated peptide antigen for CD8+ CTLs both *in vivo* and *in vitro* (Celluzzi et al., *J. Exp. Med* 183:283 (1996)). Based on the positive results using FITC, the inventors followed a similar protocol in an attempt to induce immunity to the EG7-OVA tumor by topical administration of the well-characterized, synthetic SIINFEKL tumor-associated peptide.

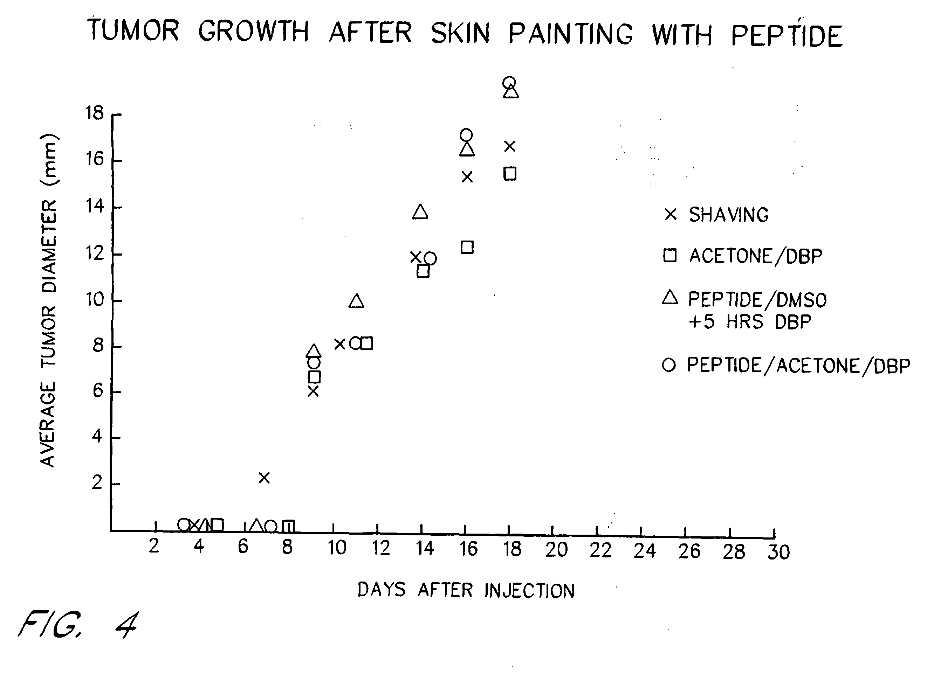

C57BU6 mice were treated as follows: 1) shaving alone; 2) topical administration of acetone and dibutylphthalate; 3) SIINFEKL (240 :g/ml) in DMSO diluted in PBS, followed in 5 hours by acetone and dibutylphthalate; and 4) SIINFEKL (240 :g/ml) in acetone and dibutylphthalate. All mice were subsequently injected subcutaneously with 5 × 10⁵ EG7-OVA cells (5-times the minimal tumorogenic dose). Tumor-specific immunity was monitored by measuring tumor size. The results shown in Figure 4, indicate that none of the treatment protocols were effective in inhibiting tumor cell growth. Since it was known from the FITC experiments that dibutylphthalate was a potent Langerhans cell migration inducer, and that SIINFEKL was readily incorporated into the MHC class I molecule K^{b} where it activated CD8+ CTLs (Celluzzi et al., *J*. *Exp*. *Med*. 183:283 (1996)), it was concluded that the SIINFEKL peptide did not get across the stratum corneum.

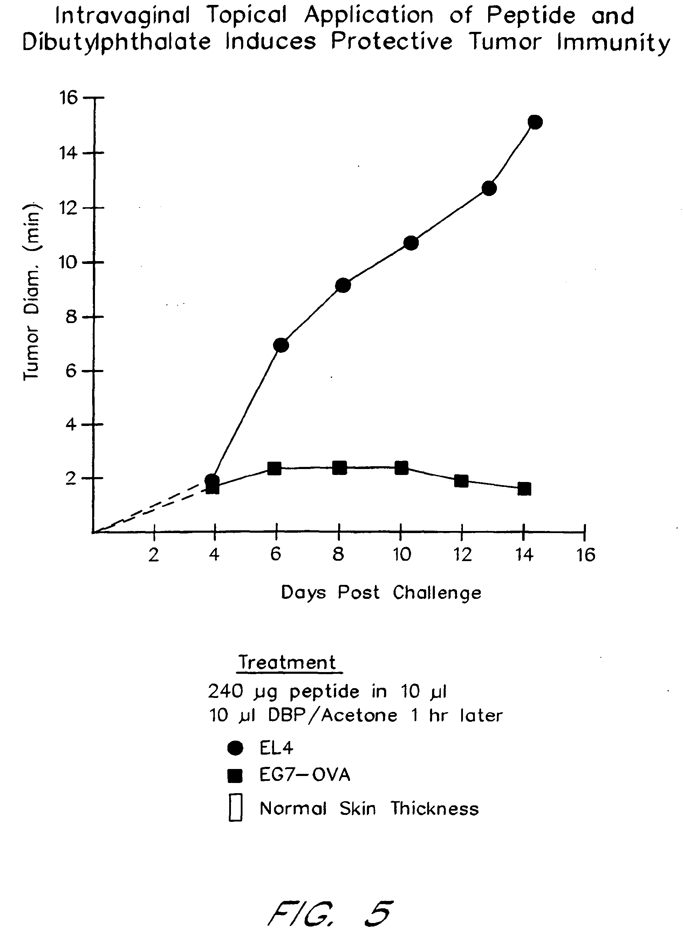

Since it was postulated that topical administration of SIINFEKL in acetone and dibutylphthalate had failed to confer tumor-specific immunity because the peptide did not penetrate the stratum corneum, the same topical vaccine was applied intravaginally. The mucous membranes lack the tough barrier posed by the stratum corneum. The results shown in Figure 5 suggested that complete protection against the tumor was induced by SIINFEKL in acetone and dibutylphthalate. The EL4 parent tumor cell line, lacking chicken ovalbumin, was used as a positive control; no immunity against the EL4 tumor cells was seen. Thus, the inventors' hypothesis regarding penetration of the stratum corneum was confirmed; the SIINFEKL peptide was not reaching the Langerhans cells underlying the stratum corneum.

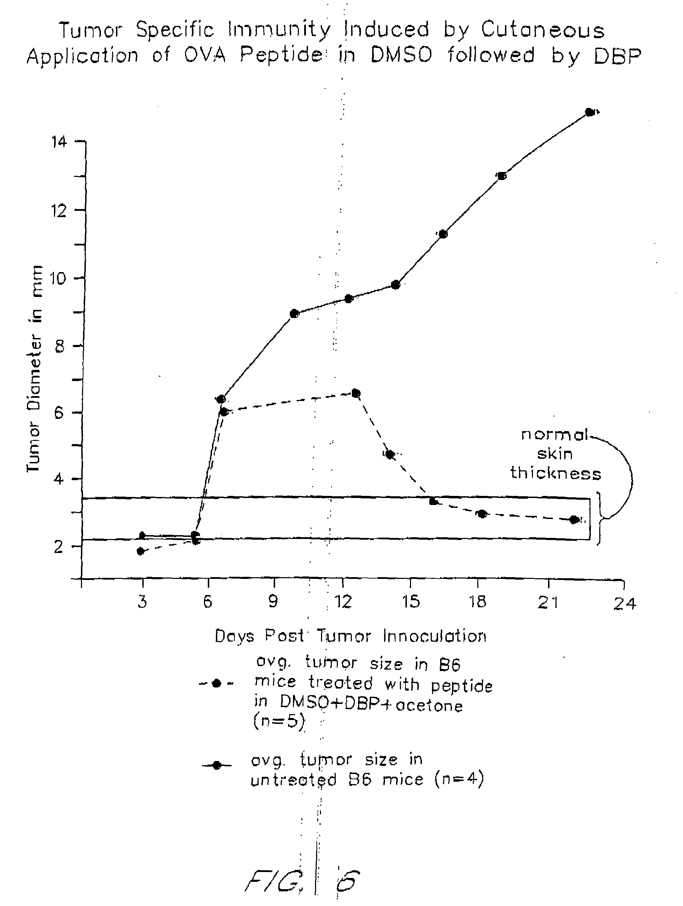

While intravaginal topical administration of tumor antigen provided an effective means of routing antigen to the Langerhans cells for subsequent induction of tumor-specific immunity, mucous membrane application sites, like the vagina, are not well-suited for convenient use and monitoring by general practitioners likely to be involved in administering the topical vaccinations of the present invention. Thus, to enhance the effectiveness of cutaneous topical administration, SIINFEKL was dissolved in DMSO and applied to the skin without dilution. Dibutylphthalate in acetone was applied to the same site 5 hr later. All mice were subsequently injected subcutaneously with 5 x 10⁵ EG7-OVA cells. The results shown in Figure 6 indicate that the peptide in DMSO was able to traverse the stratum corneum, effectively gaining access to the Langerhans cells. Tumor growth was suppressed after 6 to 9 days and even the established tumors were completely eliminated by 16 days post-inoculation. Thus, the tumor specific peptide, SIINFEKL, in concentrated DMSO, penetrated the stratum corneum, was incorporated into the MHC class I molecule K^{b} on epidermal Langerhans cells, and in the presence of the migration inducer, dibutylphthalate, was effectively presented to CD8+ CTLs in the lymph nodes.

### Antigen Source

"Antigen" as used herein, includes any molecule which when administered in accordance with the present invention is capable of eliciting antigen-specific immunity. Accordingly, the antigen or a fragment thereof must be able to (1) penetrate the skin or mucous membrane, (2) interact with Langerhans cells in the epidermis or epithelium of mucous membranes, (3) associate in whole or in part with an MHC class I or II molecule on a Langerhans cell, and (4) activate T cell receptors on CD4+ or CD8+ T cells, conferring antigen-specific immunity. More particularly, the topical vaccination method of the present invention is directed toward antigens from tumor cells, viral and bacterial pathogens and parasites.

Small peptide antigens are particularly preferred, although not essential, as it is easier to get smaller peptides (about 3.20 amino acid residues) across the stratum corneum and into the epidermis than larger proteins. Furthermore, properly sized peptides, like SIINFEKL, are more likely to bind directly to MHC molecules on the Langerhans cell surface through the process of peptide exchange, in which the exogenously added peptides displace those endogenous peptides, which may exhibit a lower affinity for the peptide binding groove of the MHC class I or II molecules. Characteristics of peptides suitable for direct association with class I or II MHC molecules have been studied extensively. Thus, those skilled in the art would be able to predict, based upon peptide structure, sequences likely to associate with a given MHC. Peptides which bind effectively to class I MHC molecules are generally comprised of about 8-9 amino acid residues, whereas peptides which associate preferentially with the class II MHC molecules are about 11-14 amino acid residues in length. Indeed, some antigenic peptides capable of interacting directly with MHC molecules on dendritic cells are known and can be synthesized by standard techniques.

Alternatively, while specific tumor-associated antigens have been identified for many human tumors, the relevant peptides are often unknown. Nonetheless, crude acid-eluted tumor peptides can be prepared quickly and easily and have been shown to be effective inducers of tumor-specific immunity when associated with dendritic cells (Zitvogel et al., *J. Exp. Med.* 183:87-97, 1996). Thus, both known, homogeneous preparations of synthetic peptides, as well as unknown mixtures of extracted peptides, may be suitable for use in the present invention. The choice and preparation of suitable peptide antigens is well within the skill of those in the art. See below for a detailed description of specific working examples.

Non-peptide immunogenic compounds capable of induction of specific immunity are also contemplated as potential antigens in accordance with the present invention. Small non-peptide haptens may covalently bind to peptides or proteins after crossing the stratum corneum, thereby gaining access to standard antigen presentation pathways, through association with an MHC molecule. For example, the immunogenicity of FITC was shown to be due to the reactivity of its isothiocyanate group (-N-C-S) with free amino groups, permitting it to be covalently incorporated within proteins and/or peptides. Thus, non-peptide antigenic moieties, from tumor cells, pathogens, parasites, allergens, etc., may be used in practicing the topical vaccination methods of the present invention.

It should be noted that choice of the source of antigen is not critical to the invention, which is a general method for enhancing antigen-specific immunity. However, the specific immune response obtained will, of course, depend on the particular antigen employed. It is the manner of topical administration, the penetration of the stratum corneum, the induction of Langerhans cell migration and the subsequent antigen presentation by MHC-dependent pathways, which individually or in combination, exemplify the features of the disclosed invention. The fact that no limitations are placed on the selection of antigens from a wide variety of sources is consistent with use of the basic methods for inducing antigen-specific immunity against many antigens. Thus, the choice of a particular antigen and its source will depend on the application and is within the ordinary skill of those in the field of immunology.

### Penetration of the Stratum Corneum

The outer layer of the skin is designed to resist entry of foreign materials into the body. Because it is formed of densely packed layers of keratinocyte cell membranes and keratin fibrils, the stratum corneum is impermeable to most molecules of higher molecular mass, particularly those of a hydrophilic nature. Consequently, only a few drugs are administered transdermally. Such molecules are typically small and lipophilic. The transdermal delivery of peptides is ill-favored because of their hydrophilicity and generally high molecular mass. Numerous studies document the great difficulty in getting peptides through the stratum corneum (For review, see Steinstrasser and Merkle, *Pharm*. *Acta*. *Helv*. 70:3-24 (1995)). Among the approaches which have yielded some success in enhancing peptide penetration are included lipophilic vehicles, low frequency ultrasound, electroporation, iontophoresis, and intraepidermal delivery. These are considered below.

Lipophilic Solvents - lipophilic solvents like acetone, azone, and dimethylsulfoxide (DMSO) are known to penetrate the stratum corneum. As discussed above, the inventor has found that small peptides, like SIINFEKL, dissolved in DMSO cross the stratum corneum and enter the lower strata of the epidermis. Thus, the present disclosure with respect to antigen penetration enhancers, encompasses a variety of lipophilic solvents, including DMSO, and symmetrical or unsymmetrical sulfide and sulfoxides where the alkyl group contains 1 to 16 carbon atoms, as well as liposomes.

Where lipophilic penetrants, such as DMSO, are employed in the present invention to enhance peptide penetration, such solvents may be administered neat or diluted with other solutions, such as PBS. Ratios of mixtures may range from about 1:9 to about 9:1 (penetrant to diluant). Preferably, the penetration enhancer is undiluted.

Low Frequency Ultrasound. Mitragotri et al. reported that even large protein molecules, such as insulin (mw 6,000), IFN-( (mw 17,000), and erythropoietin (mw 48,000) could be coaxed into crossing the stratum corneum, at about 12% efficiency, using low frequency ultrasound (Mitragotri et al., *Science* 269:850-853 (1995) ). Ultrasound works on skin by non-thermal, cavitational effects, creating micro-bubbles that expand and contract in the stratum corneum, resulting in a transient increase in permeability. Cavitation occurs at much lower frequency sound waves (i.e. about 20 kHz) than traditional diagnostic imaging (2-12 Mhz); both applications requiring an intensity of approximately 0.2 W/cm².

Electroporation - Vanbever et al. disclosed that about 10% of the small (mw 267) molecule, metoprolol, could be transported across the skin during 4 hr after 5 single 450 V pulses (Vanbever et al., *Pharm. Res.* 11:1000-1003 (1994)). Electroporation has been used extensively for permeabilization of cell membranes for the purpose of getting DNA into cells. Transdermal transport of peptides may be accomplished by localized exposure of the skin to high intensity electric field pulses, which may create transient aqueous pores in the lipid bilayers.

Iontophoresis - Bodde et al. found that only about 0.4% per hour of vassopressin (mw 1084) crossed the stratum corneum (Bodde et al., *Biochem*. *Soc*. *Trans*. 17:943-945 (1990)). Iontophoresis involves exposing the skin to low intensity current. Polar molecules move in response to the current. However, transport is believed to occur via glands or hair follicles which project through the epidermis down into the dermis. Consequently, the method may not be ideally suited for providing access of the epidermal Langerhans cells to the permeating peptides. Nonetheless, the method is encompassed within the present invention.

Intraepidermal Delivery - Certainly, the stratum corneum can be penetrated using sharp instruments. Thus, intraepidermal injections using traditional beveled needles and syringes is possible. Similarly, introduction of the antigenic agents into the epidermis using pronged instruments, like those used for administration of the tine test, is also possible. Such invasive physical methods may solve the penetration problem and may even provide concomitant migratory signals to the Langerhans cells. However, such methods may also defeat some of the key advantages of the present invention, such as the ease of topical application without the need for sterile instruments or highly skilled health care personnel. Furthermore, the use of invasive procedures are always accompanied by increased risks of infection.

### Induction of Langerhans Cell Migration

While effectively bringing antigens into contact with the Langerhans cells in the epidermis is necessary for practice of the present topical vaccination method, it is not by itself sufficient to induce the strong antigen-specific immunity desired for a vaccination procedure. Indeed, it is the second step, the induction of Langerhans cell migration, which remained obscure for so long until the present disclosure. Although the phenomenon of Langerhans cell migration from the epithelium to the lymph nodes has been well known for many years, the complex regulatory pathways which control Langerhans cell migration and differentiation *in vivo* are not well understood. Moreover, there are apparently a number of commonly held misconceptions regarding Langerhans cell migration. For example, some investigators believe that the spontaneous and/or continuous migration of Langerhans cells is sufficient for induction of antigen-specific immunity (see e.g. Matsuno et *al*., *J*. *Exp*. *Med*. 183:1865 (1996)). Others in the field believe the fact that contact sensitizing antigens are capable of inducing Langerhans cell migration by themselves, indicates that a separate induction stimulus is not needed for any antigens (see e.g. Udey, *Clin*. *Exp*. *Immunol*. 107:6 (1997)). Accordingly, scientists using FITC in acetone and dibutylphthalate to study Langerhans cell migration believe that it is the antigen (FITC) which induces migration (see e.g. Tang et al., *J*. *Immunol*. 88:284 (1996)). In summary, the state of the art in this field today, contemplates neither the existence of exogenous inducers of Langerhans cell migration, nor a role for such inducers in enhanced antigen-specific immunity.

At present, inquiry into regulation of Langerhans cell migration focuses on the production cytokines and chemokines by a variety of cells, which appear to modulate Langerhans cell migration and maturation *in vitro*. For instance, granulocyte-macrophage colony-stimulating factor (GM-CSF) and interteukin-1*α* (IL-1α) may mediate *in vitro* maturation (Larregina et al., *Immunology* 87:317-325, 1996). Tumor necrosis factor-α (TNF-α) is involved in Langerhans cell migration (Banchereau and Steinman, *Nature* 392-245 (1998)). Other factors implicated in the control of various aspects of Langerhans cell maturation and/or migration include MIP-1a, IL-4, G-protein-coupled receptors for the calcitonin-gene related peptide, C5a and other chemokines (Banchereau and Steinman, *Nature* 392:245 (1998)). Thus, while many possible signals and signaling pathways have been identified, the regulatory scheme remains obscure.

Furthermore, the elaborated signals and pathways discussed above tend to focus on the "normal" response to traumatic breaches in the epidermis. Exogenous inducers of Langerhans cell migration have not been considered before this work by the inventor. Indeed, researchers had not even recognized that stimulation of Langerhans cell migration was a critical step in the therapeutic induction of antigen-specific immunity.

Compounds with the capacity to induce Langerhans cell migration may be represented by the general formula: 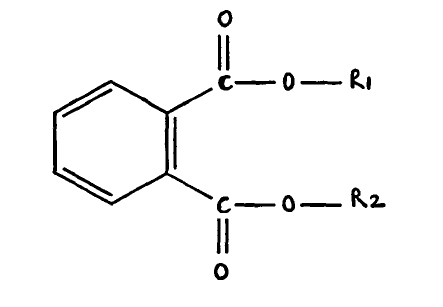 wherein R₁ and R₂ are independently, alkyl or aryl side chains containing from 1 to 16 carbon atoms. The aryl moieties are preferably substituted or unsubstituted benzyl or phenyl moieties. In one particularly preferred embodiment, the R₁ and R₂ groups are identical alkyl moieties from 2 to 6 carbons, such as dibutylphthalate, wherein R₁ and R₂ are (CH₂)₃-CH₃.

Compounds which are useful as inducers of Langerhans cell migration may be screened using the standard FITC methods described for the data presented in Figures 1-3. C57BL6 mice are shaved on their abdomens and painted with 100 µl test solution, containing 5 mg/ml FITC in a 50150 (v/v) mixture of acetone and the migration inducer (e.g. dibutylphthalate). After 2 days, the draining inguinal lymph node is removed and the total number of dendritic cells (both FITC+ and FITC-) is determined by flow cytometry and MHC class II immunoassay.

Specific compounds encompassed within the present invention for induction of Langerhans cell migration are provided below:

| Abbreviation | Compound |
|---|---|
| DBP | dibutylphthalate |
| DBT | dibutyl-D-tartrate |
| DET | N,N-diethyl-toluamide |
| DBF | dibutylfumarate |
| DEHF | di(2-ethylhexyl)fumarate |
| DIOM | diisooctylmaleate |
| DEHM | di(ethythexyl)maleate |
| DIOF | disooctylfumarate |
| BA | benzoic acid |
| BC | benzalkonium chloride |
| C | camphor |
| BM | bihenylmaleate |
| DOP | dioctylphthalate |
| DBM | dibutylmaleate |
| DOM | dioctylmaleate |
| DBS | dibutylsuccinate |
| DOS | dioctylsuccinate |
| DNP | dinonylphthalate |
| DINP | diisononylphthalate |
| DMP | dimethylphthalate |
| DEP | diethylphthalate |
| DPP | dipropylphthalate |
| DphP | diphenylphthalate |
| DBBP | dibenzylbutylphthalate |
| DMEP | diethylmethylphthalate |

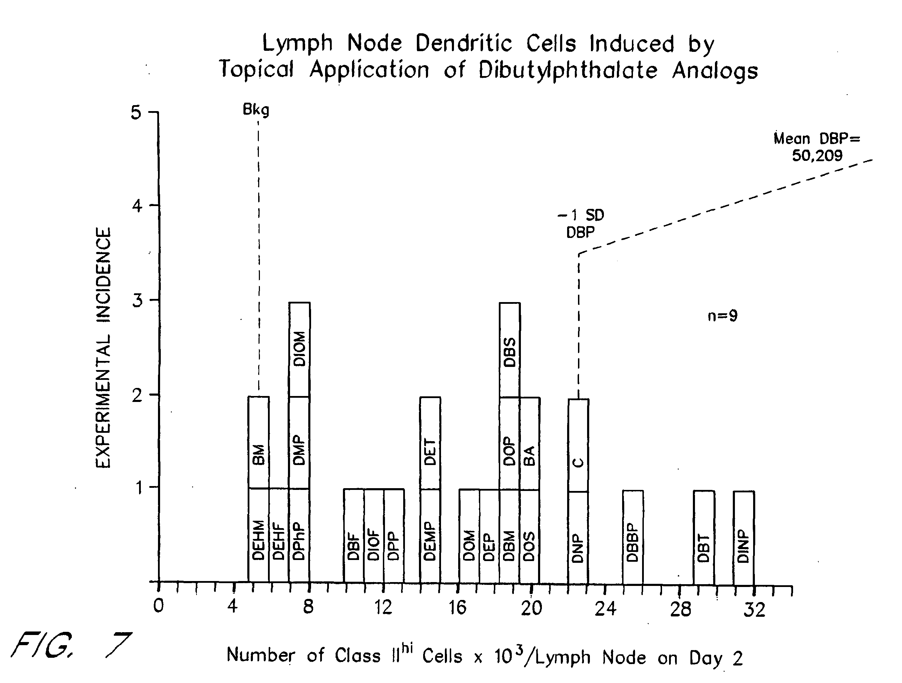

Test results are shown in Figure 7. Total MHC class II^{th} cells were increased over background for most compounds tested. DNP, C, DBBP, DBT, and DINP were within 1 standard deviation of the positive control, dibutylphthalate (DBP).

Besides the chemical inducers of Langerhans cell migration, the inventor has found that low frequency ultrasound also exhibits positive results in the FITC migration screening procedure described above. Thus, in one embodiment of the present invention, low frequency ultrasound may be employed both as a penetrant and/or as an inducer of Langerhans cell migration.

The peptides/antigens used in accordance with the present invention may generally be applied topically to epidermal or epithelial sites in a dose range of approximately 1 *µ*g/ml to about 100 mg/ml. Preferably, peptide antigens may be administered within the dose range of 1 mg/ml to 10 mg/ml. Topical administration to the skin may include from 0.01 to 1 ml application volumes, preferably about 0.05 to 0.5 ml. Generally, a lesser volume may be applied when vaginal or other mucous membrane route of administration is selected. Routes of administration may be selected from any epidermal and/or mucous membrane sites including, the skin, intravaginal, rectal, aerosol delivery to the airways and lungs, and possibly administration to the GI tract for some antigens. Where chemical inducers of Langerhans cell migration are applicable, the chemical inducer may be given neat or mixed with an organic solvent, such as acetone, in a range of ratios from about 1:9 to about 9:1 (inducer to solvent), preferably in a range of ratios from about 3:7 to about 7:3, and most preferably at a ratio of approximately 1:1.

In some instances, it may be advantageous, merely to enhance Langerhans cell migration, without specifically administering any antigen. For example, where an individual has a skin cancer, such as melanoma or basal cell carcinoma, it may be useful in enhancing an immune response against the tumor to administer an inducer of Langerhans cell migration to sites surrounding the tumor, before surgery. Alternatively, topical administration may follow excision and subsequent chemotherapy and/or radiation.

## Claims

1. Use of a kit of parts containing:
- an antigen;
- means for enhancing penetration of said antigen through the epidermis or epithelium of a mammal, said means being a lipophilic solvent and/or a physical stimulus selected from the group consisting of low frequency ultrasound and an electrical field;
- an inducer of Langerhans cell migration, comprising a compound of the form 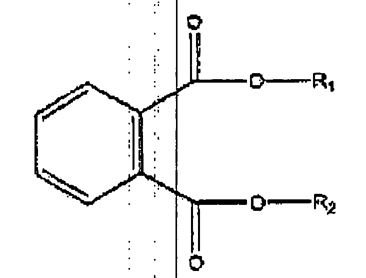
wherein R₁ and R₂ are independently alkyl or aryl side chains containing from 1 to 16 carbon atoms
for the manufacture of a medicament for enhancing an immune response against said antigen in a mammal by topical administration.

2. Use according to claim 1, wherein said compositions are to be administrated to an epidermal or mucous membrane site on said mammal.

3. Use according to claim 1 or 2, wherein said antigen is a peptide of about 3-20 amino acid residues in length.

4. Use according to claim 3, wherein said peptide is from 8-14 amino acids in length.

5. Use according to claim 1 or 2, wherein said peptide is to be administered at a concentration in a range of about 1 µg/ml to about 100 mg/ml.

6. Use according to claim 1 or 2, wherein said lipophilic solvent is selected from the group consisting of symmetrical or unsymmetrical alkylsulfides and alkylsulfoxides, wherein said alkyl group contains from 1 to 16 carbon atoms.

7. Use according to claim 1 or 2, wherein said lipophilic solvent is dimethylsulfoxide.

8. Use according to claim 1 or 2, wherein said inducer of Langerhans cell migration is selected from the group consisting of dibutylphthalate, dioctylphthalate, dinonylphthalate, diisononylphthalate, dimethylphthalate, diethylphthalate, dipropylphthalate, diphenylphthalate, dibenzylbutylphthalate and diethylmethylphthalate.

9. Use according to claim 1 or 2, wherein said inducer of Langerhans cell migration is low frequency ultrasound.

10. Use according to claim 1 or 2, wherein said inducer of Langerhans cell migration is dibutylphthalate.

11. Use according to claim 1 or 2, wherein said lipophilic solvent is selected from the group consisting of symmetrical or unsymmetrical dialkyl ketones, wherein said alkyl group contains from 1 to 16 carbon atoms.

12. Use according to claim 11, wherein said lipophilic solvent is acetone.

13. Use according to claim 1, wherein said electrical field is adapted for electroporation or iontophoresis or combinations thereof.

14. Use according to any one of the preceding claims, wherein the antigen is from a source selected from the group consisting of tumors, viruses, bacteria and parasites.

15. Use of an effective amount of an inducer of Langerhans cell migration for the manufacture of a medicament for enhancing an immune response in a mammal by topical administration of said inducer of Langerhans cell migration, which inducer is selected from the group consisting of dibutylphthalate, dibutyl-D-tartrate, N,N-dietyl-toluamide, dibutylfumarate, di(2-ethylhexyl)fumarate, diisooctylmaleate, diethylexylmaleate, diisooctylfumarate, benzoic acid, benzalkoniumchloride, bihenylmaleate, dioctylphthalate, dibutylmaleate, dioctylmaleate, dibutylsuccinate, dioctylsuccinate, dinonylphtalate, diisononylphthalate, dimethylphthalate, diethylphthalate, dipropylphthalate, diphenylphthalate, dibenzylbutylphthalate and diethylmethylphthalate and camphor.

## Patentansprüche

1. Verwendung der Teile eines Kits enthaltend:
- Ein Antigen;
- Mittel zum Erhöhen der Penetration des Antigens durch die Epidermis oder das Epithel von einem Säuger, wobei die Mittel ein lipophiles Lösemittel und/oder ein physikalischer Reiz, ausgewählt aus der Gruppe bestehend aus niederfrequentem Ultraschall und einem elektrischen Feld, sind;
- Mittel zum Induzieren der Wanderung von Langerhanszellen, umfassend eine Verbindung der Form 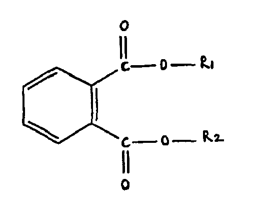
wobei R₁ und R₂ unabhängig für Alkyl- oder Arylseitenketten stehen, enthaltend von 1 bis 16 Kohlenstoffatomen, zum Herstellen eines Medikamentes zum Erhöhen einer Immunreaktion gegen das Antigen in einem Säuger durch topische Verabreichung.

2. Verwendung nach Anspruch 1, wobei die Zusammensetzungen an einer epidermalen oder Schleimhautstelle des Säugers zu verabreichen sind.

3. Verwendung nach Anspruch 1 oder 2, wobei das Antigen ein Peptid mit einer Länge von etwa 3 bis 20 Aminosäureresten ist.

4. Verwendung nach Anspruch 3, wobei das Peptid eine Länge von 8 bis 14 Aminosäuren hat.

5. Verwendung nach Anspruch 1 oder 2, wobei das Peptid mit einer Konzentration im Bereich von etwa 1 µg/ml bis etwa 100 mg/ml zu verabreichen ist.

6. Verwendung nach Anspruch 1 oder 2, wobei das lipophile Lösemittel ausgewählt ist aus der Gruppe bestehend aus symmetrischen oder asymmetrischen Alkylsulfiden und Alkylsulfoxiden, wobei die Alkylgruppe von 1 bis 16 Kohlenstoffatome enthält.

7. Verwendung nach Anspruch 1 oder 2, wobei das lipophile Lösemittel Dimethylsulfoxid ist.

8. Verwendung nach Anspruch 1 oder 2, wobei das induzierende Mittel der Wanderung der Langerhanszellen ausgewählt ist aus der Gruppe bestehend aus Dibutylphthalat, Dioctylphthalat, Dinonylphthalat, Diisononylphthalat, Dimethylphthalat, Diethylphthalat, Dipropylphthalat, Diphenylphthalat, Dibenzylbutylphthalat und Diethylmethylphthalat.

9. Verwendung nach Anspruch 1 oder 2, wobei das induzierende Mittel der Wanderung der Langerhanszellen niederfrequenter Ultraschall ist.

10. Verwendung nach Anspruch 1 oder 2, wobei das induzierende Mittel der Wanderung der Langerhanszellen Dibutylphthalat ist.

11. Verwendung nach Anspruch 1 oder 2, wobei das lipophile Lösemittel ausgewählt ist aus der Gruppe bestehend aus symmetrischen oder asymmetrischen Dialkylketonen, wobei die Alkylgruppe von 1 bis 16 Kohlenstoffatome enthält.

12. Verwendung nach Anspruch 11, wobei das lipophile Lösemittel Aceton ist.

13. Verwendung nach Anspruch 1, wobei das elektrische Feld für Elektroporation oder Iontophorese oder Kombinationen davon angeglichen ist.

14. Verwendung nach einem der vorhergehenden Ansprüche, wobei das Antigen aus einer Quelle stammt, ausgewählt aus der Gruppe bestehend aus Tumoren, Viren, Bakterien und Parasiten.

15. Verwendung einer wirksamen Menge eines induzierenden Mittels der Wanderung von Langerhanszellen zum Herstellen eines Medikamentes zur Erhöhung einer Immunreaktion in einem Säuger durch topische Verabreichung des induzierenden Mittels der Wanderung der Langerhanszellen, wobei das induzierende Mittel ausgewählt ist aus der Gruppe bestehend aus Dibutylphthalat, Dibutyl-D-tartrat, N,N-Diethyltoluamid, Dibutylfumarat, Di(2-ethylhexyl)fumarat, Diisooctylmaleat, Diethylhexylmaleat, Diisooctylfumarat, Benzoesäure, Benzalkoniumchlorid, Bihenylmaleat, Dioctylphthalat, Dibutylmaleat, Dioctylmaleat, Dibutylsuccinat, Dioctylsuccinat, Dinonylphthalat, Diisononylphthalat, Dimethylphthalat, Diethylphthalat, Dipropylphthalat, Diphenylphthalat, Dibenzylbutylphthalat und Diethylmethylphthalat und Kampfer.

## Revendications

1. Utilisation d'une trousse en plusieurs parties contenant :
- un antigène;
- un moyen pour améliorer la pénétration dudit antigène à travers l'épiderme ou l'épithélium d'un mammifère, ledit moyen étant un solvant lipophile et/ou un stimulus physique choisi dans le groupe constitué d'un ultrason à basse fréquence et d'un champ électrique;
- un inducteur de la migration des cellules de Langerhans, comprenant un composé de formule : 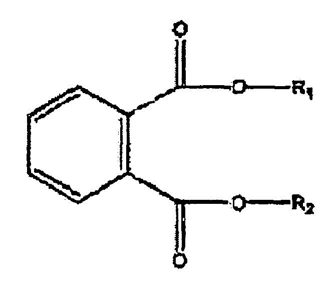
dans laquelle R₁ et R₂ représentent indépendamment des chaînes latérales alkyle ou aryle contenant 1 à 16 atomes de carbones,
pour la fabrication d'un médicament destiné à améliorer une réponse immunitaire contre ledit antigène chez un mammifère au moyen d'une administration topique.

2. Utilisation selon la revendication 1, dans laquelle lesdites compositions doivent être administrées audit mammifère au niveau d'un site de membrane d'épiderme ou de muqueuse.

3. Utilisation selon la revendication 1 ou 2, dans laquelle ledit antigène est un peptide ayant une taille d'environ 3 à 20 résidus d'acides aminés.

4. Utilisation selon la revendication 3, dans laquelle ledit peptide a une taille de 8 à 14 acides aminés.

5. Utilisation selon la revendication 1 ou 2, dans laquelle ledit peptide doit être administré à une concentration dans une plage d'environ 1 µg/ml à environ 100 mg/ml.

6. Utilisation selon la revendication 1 ou 2, dans laquelle ledit solvant lipophile est choisi dans le groupe constitué des alkylsulfures et des alkylsulfoxydes symétriques ou dissymétriques, où ledit groupement alkyle contient 1 à 16 atomes de carbone.

7. Utilisation selon la revendication 1 ou 2, dans laquelle ledit solvant lipophile est le diméthyl-sulfoxyde.

8. Utilisation selon la revendication 1 ou 2, dans laquelle ledit inducteur de la migration des cellules de Langerhans est choisi dans le groupe constitué des composés phtalate de dibutyle, phtalate de dioctyle, phtalate de dinonyle, phtalate de diisononyle, phtalate de diméthyle, phtalate de diéthyle, phtalate de dipropyle, phtalate de diphényle, phtalate de dibenzylbutyle et phtalate de diéthylméthyle.

9. Utilisation selon la revendication 1 ou 2, dans laquelle ledit inducteur de la migration des cellules de Langerhans est un ultrason à basse fréquence.

10. Utilisation selon la revendication 1 ou 2, dans laquelle ledit inducteur de la migration des cellules de Langerhans est le phtalate de dibutyle.

11. Utilisation selon la revendication 1 ou 2, dans laquelle ledit solvant lipophile est choisi dans le groupe constitué des dialkylcétones symétriques ou dissymétriques, où ledit groupement alkyle contient 1 à 16 atomes de carbone.

12. Utilisation selon la revendication 11, dans laquelle ledit solvant lipophile est l'acétone.

13. Utilisation selon la revendication 1, dans laquelle ledit champ électrique est adapté à l'électroporation ou à l'iontophorèse ou à leurs combinaisons.

14. Utilisation selon l'une quelconque des revendications précédentes, dans laquelle l'antigène provient d'une source choisie dans le groupe constitué des tumeurs, des virus, des bactéries et des parasites.

15. Utilisation d'une quantité efficace d'un inducteur de la migration des cellules de Langerhans pour la fabrication d'un médicament en vue d'améliorer une réponse immunitaire chez un mammifère par administration topique dudit inducteur de la migration des cellules de Langerhans, l'inducteur étant choisi dans le groupe constitué des composés phtalate de dibutyle, D-tartrate de dibutyle, N,N-diétyltoluamide, fumarate de dibutyle, fumarate de di(2-éthylhexyle), maléate de diisooctyle, maléate de diéthylhexyle, fumarate de diisooctyle, acide benzoïque, chlorure de bensalkonium, maléate de biphényle, phtalate de dioctyle, maléate de dibutyle, maléate de dioctyle, succinate de dibutyle, succinate de dioctyle, phtalate de dinonyle, phtalate de diisononyle, phtalate de diméthyle, phtalate de diéthyle, phtalate de dipropyle, phtalate de diphényle, phtalate de dibenzylbutyle, phtalate de diéthylméthyle et camphre.


### SEQUENCE LISTING

<110> LIDAK PHARMACEUTICALS
<120> TOPICAL IMMUNOSTIMULATION TO INDUCE LANGERHANS CELL MIGRATION
<130> P.KNOB.104/EP(WO)
<140> 98918392.6
   <147> 1998-04-20
<160> 1
<170> PatentIn Ver. 2.1
<210> 1
   <211> 8
   <212> PRT
   <213> Chicken ovalbumin
<400> 1
   Ser Ile Ile Asn Phe Glu Lys Leu
    1               5